(19) Europäisches Patentamt
European Patent Office
Office européen des brevets

(11) EP 0 973 705 B1

(12) **EUROPÄISCHE PATENTSCHRIFT**

(45) Veröffentlichungstag und Bekanntmachung des Hinweises auf die Patenterteilung:
**27.07.2005 Patentblatt 2005/30**

(21) Anmeldenummer: **98919159.8**

(22) Anmeldetag: **26.03.1998**

(51) Int Cl.[7]: **C07B 57/00**, C08F 20/54, C07D 309/30, C07D 405/06

(86) Internationale Anmeldenummer:
**PCT/EP1998/001788**

(87) Internationale Veröffentlichungsnummer:
**WO 1998/045230 (15.10.1998 Gazette 1998/41)**

(54) **CHROMATOGRAPHISCHE ENANTIOMERENTRENNUNG VON LACTONEN**

CHROMATOGRAPHIC ENANTIOMER SEPARATION OF LACTONES

SEPARATION CHROMATOGRAPHIQUE PAR ENANTIOMERES DE LACTONES

(84) Benannte Vertragsstaaten:
**AT BE CH DE DK ES FI FR GB GR IE IT LI LU MC NL PT SE**

(30) Priorität: **08.04.1997 DE 19714343**

(43) Veröffentlichungstag der Anmeldung:
**26.01.2000 Patentblatt 2000/04**

(73) Patentinhaber: **LANXESS Deutschland GmbH**
**51369 Leverkusen (DE)**

(72) Erfinder:
- **BÖMER, Bruno**
  **DECEASED (DE)**
- **LANGE, Walter**
  **D-50733 Köln (DE)**
- **GROSSER, Rolf**
  **D-51373 Leverkusen (DE)**
- **KÖHLER, Burkhard**
  **D-51373 Leverkusen (DE)**
- **MICHEL, Stefan**
  **D-51373 Leverkusen (DE)**
- **ZWEERING, Uwe**
  **D-40589 Düsseldorf (DE)**

(56) Entgegenhaltungen:

| | |
|---|---|
| **EP-A- 0 155 637** | **EP-A- 0 183 132** |
| **EP-A- 0 319 847** | **EP-A- 0 379 917** |
| **EP-A- 0 409 281** | **EP-A- 0 617 019** |
| **EP-A- 0 780 408** | **WO-A-92/14692** |
| **US-A- 4 897 490** | |

- **ARLT ET AL.: "New chiral polyamid stationary phases for chromatografic enantiomer separation" ANGEW. CHEM. INT. ED. ENG., Bd. 30, Nr. 12, 1991, Seiten 1662-1664, XP002073359**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft die Verwendung von optisch aktiven Polymeren aus N-Acryloyl-phenylalanin-neomenthylamid als solchen, in vernetzter Form und/oder in trägergebundener Form als stationäre Phasen zur chromatographischen Enantiomerentrennung von Lactonen.

In EP 379 917 werden chirale stationäre Phasen beschrieben, die sich von N-(Meth)-Acryloylaminosäure-Derivaten ableiten und die sich zur Enantiomerentrennung u.a. auch von Lactonderivaten eignen. Bevorzugt werden Perlpolymerisate als Trennphasen eingesetzt.

Weiterhin ist aus EP 617 019 bekannt, daß (±)-trans-(E)-6-[2-(2,6-Diisopropyl-4-(4-fluorphenyl)-3-methoxymethyl-pyrid-5-yl)-ethenyl]-3,4,5,6-tetrahydro-4-hydroxy-2H-pyran-2-on (Ii) chromatographisch an chiralen stationären Phasen in die Enantiomere getrennt werden kann. Dieses Lacton wird, wie in EP 617 019 und 491 226 beschrieben, in 3R,5S-(+)-Natrium-erythro-(E)-7-[4-(4-fluorphenyl)-2,6-diisopropyl-5-methoxymethyl-pyrid-3-yl]-3,5-dihydroxy-hept-6-enoat (A) überführt, das die Cholesterinbiosynthese hemmt und in Arzneimitteln zur Behandlung von Lipoproteinämie eingesetzt werden kann.

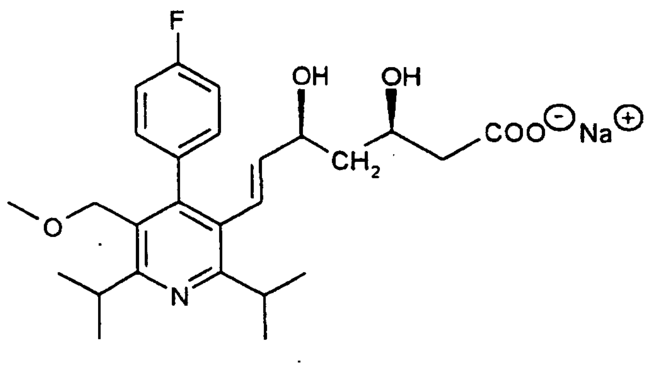

(A)

In EP 617 019 wird beschrieben, daß diese Trennung vorteilhaft durch Chromatographie an chiralen stationären Phasen durchgeführt werden kann, wie sie in EP 379 917 genannt sind. Als besonders geeignete Phasen werden dort Polymerisate aus S-Phenylalanin-d-menthylester genannt.

Es wurde nun aber überraschend gefunden, daß die Trennung von Lactonderivaten besonders effizient ist, wenn die optisch aktiven Polymere Derivate von Aminosäureamiden sind. Besonders geeignet sind Polymere aus N-Acryloyl-phenylalanin-neomenthylamid.

Die Erfindung betrifft daher die Verwendung von optisch aktiven Polymeren aus N-Acryloyl-S-phenylalanin-d-neomenthylamid oder aus dessen Enantiomer als solchen, in vernetzter Form und/oder in trägergebundener Form als stationäre Phasen zur chromatographischen Enantiomerentrennung von Lactonen der allgemeinen Formel (I)

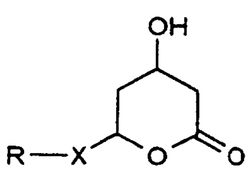

(I)

worin

R für einen organischen Rest und

X für $-CH_2-CH_2-$ oder -CH=CH- steht.

Die Lactone (bzw. die Ringöffnungsprodukte) der allgemeinen Formel (I) wirken typischerweise als HMG-CoA-Reduktase-Inhibitoren. Solche Verbindungen eignen sich beispielsweise zur Behandlung von Hyperlipoproteinämie oder Arteriosklerose.

Bevorzugte Verbindungen der Formel (I) sind HMG-CoA-Reduktase-Inhibitoren, deren Rest R ein Derivat eines aromatischen oder teilweise gesättigten Carbocyclus mit 6 oder 12 Kohlenstoffatomen, ein Indol-Derivat, ein Pyridin-Derivat, ein Pyrrol-Derivat oder ein Ethen-Derivat ist.

Besonders bevorzugte Beispiele hierfür sind die folgenden Verbindungen:

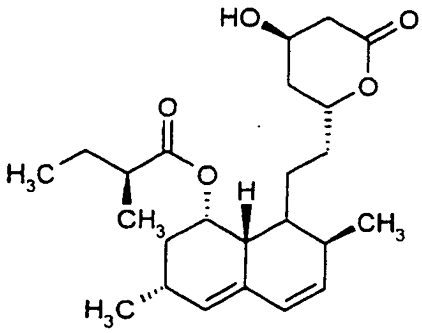

(Ia)

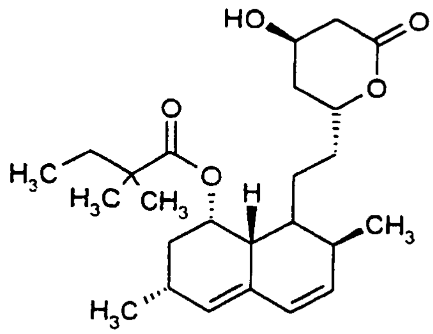

(Ib)

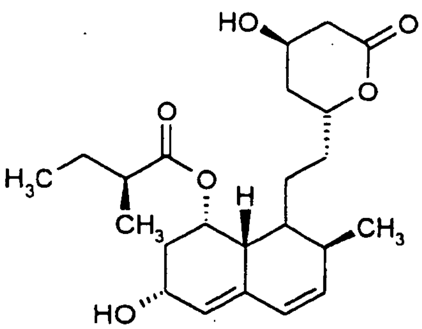

(Ic)

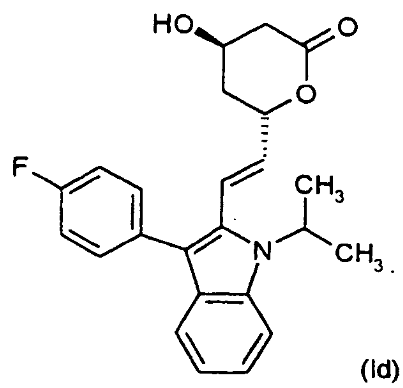

(Id)

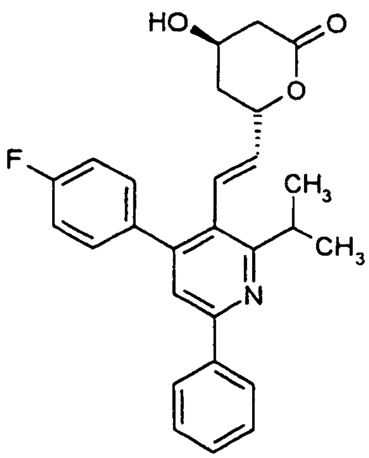

(Ie)

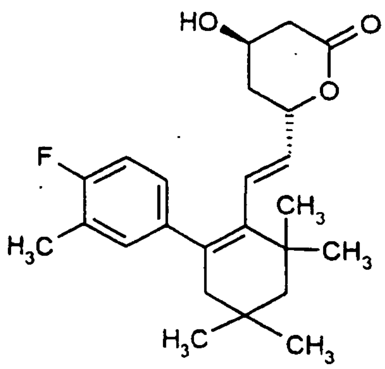

(If)

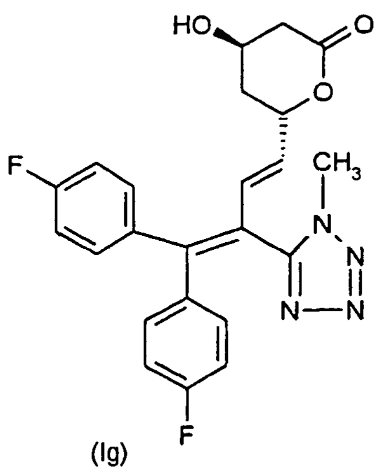

(Ig)

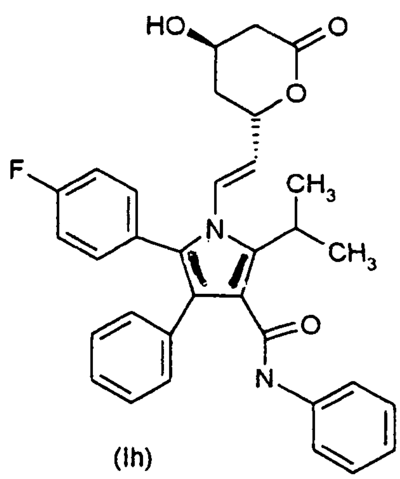

(Ih)

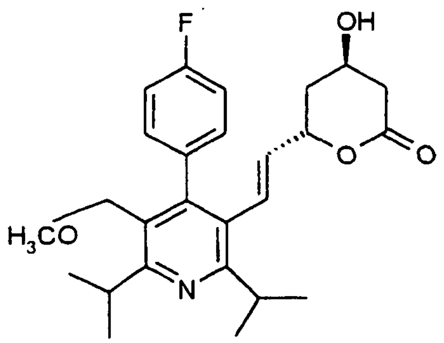

und

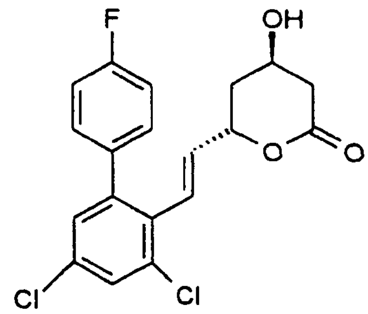

(Ii) (Ik)

Gemäß einer ganz besonders bevorzugten Ausführungsform betrifft die Erfindung die Verwendung von optisch aktiven Polymeren aus N-Acryloyl-S-phenylalanin-d-neomenthylamid oder aus dessen Enantiomer als solchen, in vernetzter Form und/oder in trägergebundener Form als stationäre Phasen zur chromatographischen Enantiomerentrennung von (±)-trans-(E)-6-[2-(2,6-Diisopropyl-4-(4-fluorphenyl)-3-methoxymethyl-pyrid-5-yl)-ethenyl]-3,4,5,6-tetrahydro-4-hydroxy-2H-pyran-2-on.

Weiterhin betrifft die Erfindung auch ein Verfahren zur chromatographischen Enantiomerentrennung von Lactonverbindungen der allgemeinen Formel (I), dadurch gekennzeichnet, daß man das entsprechende Enantiomerengemisch unter Verwendung einer geeigneten mobilen Phase mittels eines optisch aktiven Polymers aus N-Acryloyl-S-phenylalanin-d-neomenthylamid oder aus dessen Enantiomer als chiraler stationärer Phase in die Enantiomere trennt, wobei das optisch aktive Polymer als solches, in vernetzter Form und/oder in trägergebundener Form eingesetzt wird.

Weiter oben wurde bereits erläutert, welche Lactonverbindungen der allgemeinen Formel (I) bevorzugt nach diesem Verfahren in die Enantiomere getrennt werden können.

Die erfindungsgemäß verwendeten chiralen stationären Phasen leiten sich von N-Acryloyl-S-phenylalanin-d-neomenthylamid oder von seinen Enantionmeren, N-Acryloyl-R-phenylalanin-l-neomenthylamid, ab. Bevorzugt sind die von N-Acryloyl-S-phenylalanin-d-neomenthylamid abgeleiteten Phasen.

N-Acryloyl-S-phenylalanin-d-neomenthylamid oder sein Enantiomer kann nach bekannten Verfahren, z.B. wie in US 5 274 167/EP 379 917 beschrieben, hergestellt werden.

Das erfindungsgemäß verwendete optisch aktive N-Acryloyl-phenylalanin-neomenthylamid-Polymere wird vorzugsweise in Form von vernetzten unlöslichen, aber quellbaren Polymerisaten oder in an feinteilige anorganische Trägermaterialien gebundener Form eingesetzt. Es kann auch als lineares, in geeigneten organischen Lösungsmitteln lösliches Polymer hergestellt werden. Es ist ferner möglich 0,1 bis 60, vorzugsweise 0, 1 bis 20 Mol-% copolymerisierbarer, nicht chiraler Monomere in das Polymere einzubauen.

Die vernetzten Polymerisate liegen vorzugsweise in Form feinteiliger Perlen mit 5 bis 200 μm, vorzugsweise 10-100 μm, Teilchendurchmesser vor. Sie können in an sich bekannter Weise durch Polymerisation unter Zusatz eines geeigneten Vernetzers hergestellt werden, beispielsweise wie in US 5 274 167/EP 379 917 beschrieben.

Durch die Art und Menge des Vernetzers (der Vernetzer) läßt sich der Quellungsgrad der (Perl)Polymerisate einstellen.

Bei der praktischen Verwendung haben sich (Perl)Polymerisate mit einem Quellungsgrad (Q) von 1.1 bis 10, bevorzugt 2.0 bis 7.0 bewährt.

$$Q= \frac{\text{Harzvolumen (gequollen)}}{\text{Harzvolumen (ungequollen)}}$$

Besonders bevorzugt wird als stationäre Phase das Polymer aus N-Acryloyl-S-phenylalanin-d-neomenthylamid bzw. dessen Enantiomer in an feinteilige anorganische Trägermaterialien, vorzugsweise an Kieselgel, gebundener Form eingesetzt.

Die radikalische Belegung der Kieselgele mit polymerisationsaktiven Gruppen und die Polymerisation kann nach an sich bekannten Methoden erfolgen.

Das optisch aktive Polymer kann beispielsweise auf das Kieselgel aufgezogen werden, indem es physikalisch adsorbiert oder kovalent fixiert wird. Letzteres kann geschehen, indem die Kieselgeloberfläche mit polymerisierbaren Gruppen belegt wird und anschließend eine Copolymerisation mit dem optisch aktiven Monomer vorgenommen wird. Breit anwendbar ist auch die Polymerisation des optisch aktiven Monomeren in Gegenwart von Kieselgel-Diolphasen, die mit (Meth)acrylsäure verestert wurden. Geeignete Verfahren sind beispielsweise in EP 379 917 und EP 282 770 beschrieben.

Gemäß einem besonders bevorzugten Verfahren wird das Kieselgel zunächst mit Mercaptogruppen (SH-Einheiten) belegt und anschließend unter Polymerisationsbedingungen mit dem optisch aktiven Monomer umgesetzt (vgl. unsere ebenfalls anhängige europäische Patentanmeldung, Anm.Nr. 96 119 045.1).

Das an der Oberfläche mit SH-Einheiten modifizierte Kieselgel erhält man zweckmäßigerweise dadurch, daß das Ausgangsmaterial mit einer Verbindung umgesetzt wird, die mindestens eine Mercaptogruppe enthält. Geeignete Derivatisierungsreagenzien sind im Prinzip (V.R. Meyer, Praxis der Hochleistungsflüssigchromatographie, Salle + Sauerländer, 6. Aufl. 1990, S 79 ff. und dort zitierte Literatur) bekannt; sie haben die allgemeine Form Q-L-SH, wobei Q für eine reaktive Gruppe steht, die mit den OH-Gruppen des Kieselgels reagieren kann und L für eine unter den entsprechenden Bedingungen inerte Spacergruppe steht, die für den notwendigen Abstand zwischen Kieselgel und SH-Gruppe sorgt.

Bevorzugt erfolgt die Belegung von Kieselgelen durch Umsetzung eines nicht modifizierten Kieselgels mit einem Silan der Form $Z_1Z_2Z_3$Si-L-SH, wobei $Z_1$, $Z_2$ und $Z_3$ unabhängig voneinander für Alkyl mit bis zu 4 Kohlenstoffatomen, Halogen, Alkoxy mit bis zu 4 Kohlenstoffatomen oder Hydroxyl stehen und L für eine gegebenenfalls substituierte Alkylenkette mit bis zu 7 Kohlenstoffatomen steht.

Die Umsetzung kann basenkatalysiert oder im sauren Medium erfolgen. Üblicherweise wird das Kieselgel im Verhältnis Funktionalisierungsreagenz zu Kieselgel 1:20 bis 1,2:1 umgesetzt. Es resultieren Kieselgele, die 0,1 % bis 5 %, besonders bevorzugt 0,5 % bis 3 % Schwefel in Form von SH-Gruppen enthalten und in denen das optisch aktive Polymer an die Mercaptogruppen des modifizierten Kieselgels gebunden ist.

Gemäß den oben angegebenen Verfahren wird in einem ersten Schritt das Kieselgel mit einer polymerisationsfähigen oder pfropfbaren Mono-, Di- oder Trialkoxy- oder Mono-, Di- oder Trichlorsilanverbindung belegt, vorzugsweise mit Mercaptopropyltrimethoxysilan, Mercaptopropyl-triethoxysilan, Mercaptopropyl-methyl-dimethoxysilan, Bis (3-Trimethoxysilylpropyl)tetrasulfon, Thiocyanatopropyltrimethoxysilan, Thiocyanatopropyltriethoxysilan, Bis (3-Triethoxysilylpropyl)tetrasulfon, Trimethoxyvinylsilan, Triethoxy-vinylsilan, Trichlorvinylsilan, Dimethoxy-methylvinylsilan, Dichlor-methyl-vinylsilan, Chlormethylvinylsilan, Methoxy-dimethyl-vinylsilan, Methacryloyloxypropyl-trimethoxysilan, Methacryloyloxypropyl-triethoxysilan, Glycidyloxypropyl-triethoxysilan, Glycidyloxypropyl-trimethoxysilan, besonders bevorzugt mit den oben genannten Mercaptosilanen. Ganz besonders bevorzugt sind Mercaptopropyl-trimethoxysilan und Mercaptopropyltriethoxysilan. Die polymerisationsfähige oder pfropfbare Mono-, Di- oder Trialkoxy- oder Mono-, Di- oder Trichlorsilanverbindung wird in Mengen von 5 bis 120 Gew.-% bezogen auf das Kieselgel zugesetzt. Bevorzugt werden von den Mercaptosilanen 5 bis 15 Gew.-% und von den Vinylsilanen 80 bis 120 Gew.-% eingesetzt.

Als zweiter Schritt erfolgt die radikalische Polymerisation durch Zugabe des N-Acryloyl-phenylalanin-neomenthylamids in Mengen von 10 bis 100 Gew.-%, bevorzugt 30 bis 60 Gew.-%, bezogen auf das belegte Kieselgel zu der Suspension des belegten Kieselgels in einem Lösungsmittel, wie z.B. Toluol, Benzol, Chlorbenzol, Chloroform, Isopropanol, n-Butanol, Cyclohexanol, MIBK, Essigester oder Dioxan, wobei bezogen auf die Menge an N-Acryloylaminosäure-neomenthylamid 0,5 bis 10 Gew.-%, bevorzugt 1 bis 5 Gew.-%, eines Radikalkettenstarters, wie z.B. Azobisisobutyronitril oder Benzoylperoxid zugesetzt werden und 1 bis 24 h auf 56 bis 110°C erhitzt wird.

Die erfindungsgemäß verwendeten Kieselgele sind sphärisch oder irregulär und haben einen durchschnittlichen Teilchendurchmesser von 1 bis 200, bevorzugt 5 bis 50 μ. Sie sind kommerziell erhältlich, z.B. bei den Firmen Macherey und Nagel, Merck, YMC oder Akzo.

Als Fließmittel (mobile Phase) für die Trennung des Racemats sind alle üblichen organischen Lösungsmittel und Lösungsmittelgemische geeignet, die das Racemat zu lösen vermögen. Falls das optisch aktive Polymer chemisch an das Kieselgel gebunden ist, gibt es für das zu verwendende organische Lösungsmittel keine durch die chirale stationäre Phase bedingte Einschränkung, da das Polymer nicht ausgewaschen werden kann.

Beispielhaft seien hier als Fließmittel genannt: Kohlenwasserstoffe wie Benzol, Toluol, Xylol, Pentan, Hexan, Heptan, Ether wie Diethylether, t-Butylmethylether, Dioxan, Tetrahydrofuran, Halogenkohlenwasserstoffe wie Dichlormethan, Trichlormethan, Chlorbenzol, Alkohole wie n-Butanol, n-Propanol, i-Propanol, Ethanol, Methanol, Aceton, Methylethylketon, Acetonitril, Essigester, Dimethylformamid sowie Gemische der vorstehend genannten Lösungsmittel. Besonders bevorzugte Fließmittel sind Gemische von Toluol mit Tetrahydrofuran im Verhältnis 10:1 bis 1:10, bevorzugt 5:1 bis 1:5.

Die Trennungen werden in der Regel unter den üblichen Bedingungen flüssigkeitschromatographischer Trennungen durchgeführt. Sie können sowohl im analytischen als auch im präparativen Maßstab durchgeführt werden.

Eine effiziente Trennung der Verbindungen der allgemeinen Formel (I) gelingt an einer einzelnen Säule, die

mit einem Kieselgel gefüllt ist, an das ein optisch aktives Polymer aus N-Acryloyl-(S)-phenylalanin-d-neomenthylamid bzw. aus dessen Enantiomer gebunden ist. Besonders vorteilhaft ist die Trennung, wenn man diese nicht im Batch-Betrieb an einer einzelnen Säule, sondern in einem kontinuierlichen Prozeß einsetzt, der nach dem Simulated Moving Bed-Verfahren, wie z.B. in EP 586 385 beschrieben, konzipiert ist.

Die Fähigkeit der Polymerisate zur Racemattrennung wird durch die Kapazitätsverhältnisse ($k_{1(2)}'$-Werte) für die beiden Enantiomeren (1) und (2) und den daraus resultierenden Enantioselektivitätswert $\alpha$ ausgedrückt. Diese chromatographischen Parameter sind wie folgt definiert:

$$\text{Kapazitätsverhältnis } K_{1(2)}' = (t_{1(2)}-t_{(0)})/t_{(0)}$$

und

$$\text{Enantioselektivitätswert} = \alpha = k_2'/k_1'$$

$t_0$ = xTotzeit der Säule

$t_{1(2)}$ = Retentionszeit des zuerst eluierenden Enantiomers 1 bzw. des später eluierenden Enantiomers 2

## Beispiele

### Beispiel 1

Man versetzt die Suspension von 300 g eines getrockneten irregulären Kieselgels mit einem durchschnittlichen Teilchendurchmesser von 10 µ (Polygosil 100/10 µ der Fa. Macherey & Nagel) in 3 l Toluol mit 30 g Mercaptopropyltrimethoxysilan, 9 g p-Toluolsulfonsäure und 2,4 ml Wasser und erhitzt 8 h unter Rückfluß. Man saugt über einer Fritte ab, wäscht mit Methylenchlorid, mit Methylenchlorid-Methanol 1:1 und noch zweimal mit Methylenchlorid und trocknet 24 h im Hochvakuum.

### Beispiel 2

Man legt 3 g des belegten Kieselgels aus Beispiel 1 in 12 ml Toluol vor, gibt 1,2 g N-Acryloyl-L-phenylalanin-d-neomenthylamid und 20 mg Azobisisobutyronitril hinzu und erhitzt 12 h auf 60°C. Dann werden 0,2 g 2,2-Methylen-bis-6,6-dicyclohexyl-4,4-methylphenol und 3 ml Bistrimethylsilylacetamid zugegeben und 4 h unter Rückfluß gekocht. Man saugt über eine G4-Fritte ab und wäscht mit Methylenchlorid, Methanol-Methylenchlorid 1:1, Toluol, Isopropanol und nochmals mit Methylenchlorid und trocknet 10 h im Hochvakuum.

Man erhält 3,21 g eines Kieselgels mit einem Stickstoffgehalt (bestimmt durch Elementaranalyse) von 1,6 %, entsprechend einer Polymerbelegung von 20,3 Gew.-%.

### Beispiel 3 (Trennbeispiel)

Die an Kieselgel gebundenen Polymerisate (hergestellt wie in Beispiel 1 und 2 beschrieben) wurden in Stahlsäulen (Innendurchmesser 4,6 mm; Länge 250 mm) eingesetzt. Eluiert wurde mit einem Toluol-Tetrahydrofuran-(3:1) -Gemisch. Die Fließmittelgeschwindigkeit betrug 1 ml/min.

Racemat: (±)-trans-(E)-6-[2-(2,6-Diisopropyl-4-(4-fluorphenyl)-3-methoxymethyl-pyrid-5-yl)-ethenyl]-3,4,5,6-tetrahydro-4-hydroxy-2H-pyran-2-on (Ii)

relative Kapazität $k_1'$: 0,47

relative Kapazität $k_2'$: 2,73

Enantioselektivität $\alpha$: 5,82

## Patentansprüche

**1.** Verwendung von optisch aktiven Polymeren aus N-Acryloyl-S-phenylalanin-d-neomenthylamid oder aus dessen Enantiomer als solchen, in vernetzter Form und/oder in trägergebundener Form als stationäre Phasen zur chromatographischen Enantiomerentrennung von Lactonen der allgemeinen Formel (I)

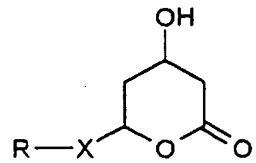

(I)

worin

R für einen organischen Rest und

X für $-CH_2-CH_2$ oder $-CH=CH-$ steht.

**2.** Verwendung von optisch aktiven Polymeren gemäß Anspruch 1 zur chromatographischen Enantiomerentrennung eines Lactons, ausgewählt aus der Gruppe bestehend aus:

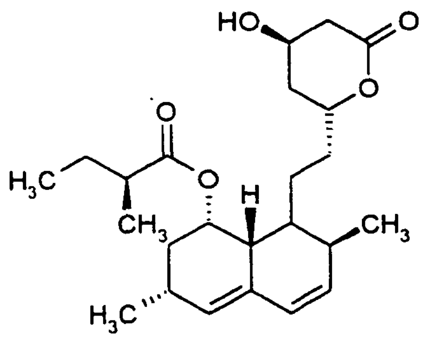

(Ia)

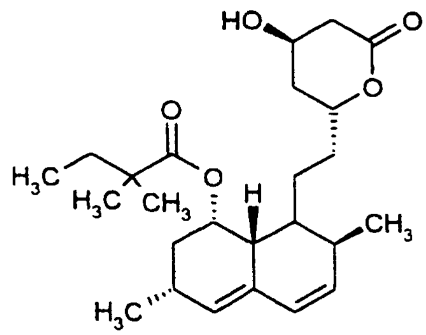

(Ib)

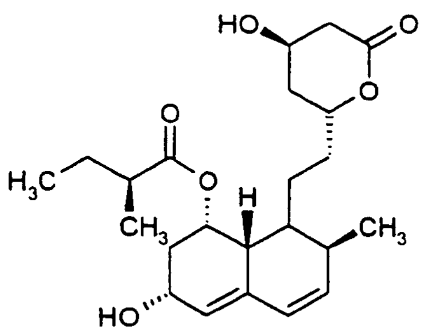

(Ic)

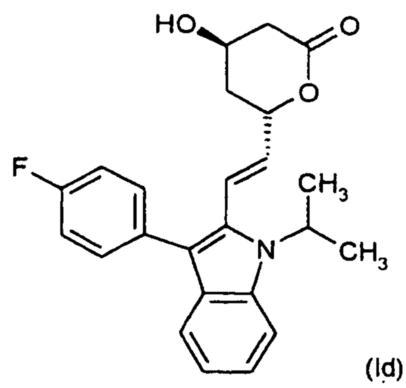

(Id)

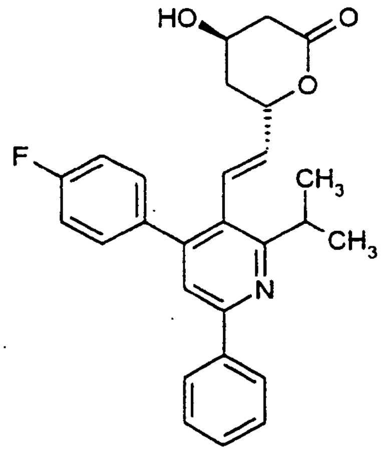

(Ie)

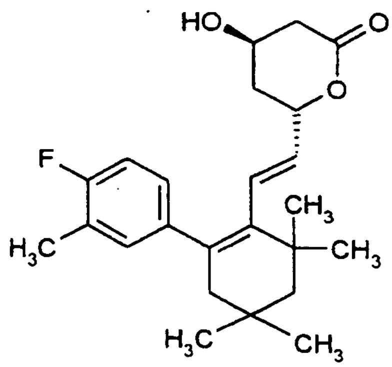

(If)

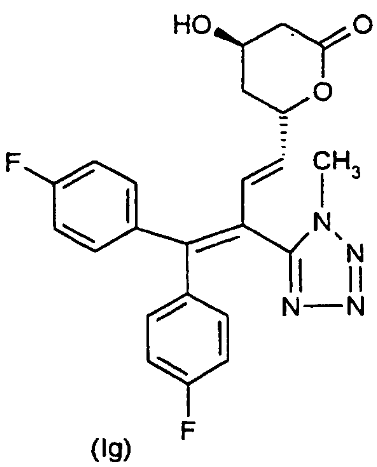

(Ig)

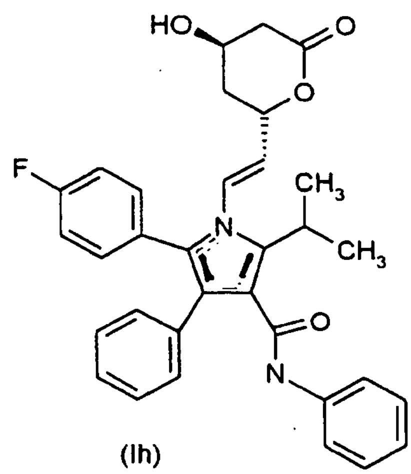

(Ih)

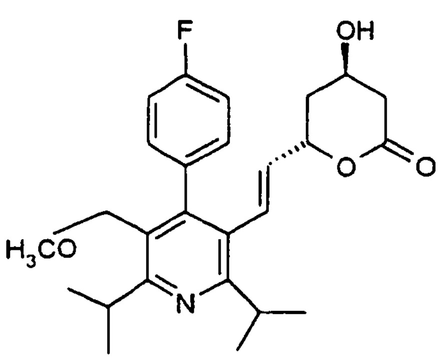

und

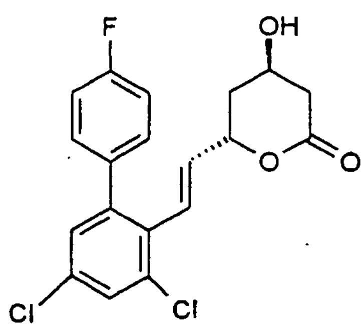

(Ii)

(Ik)

3. Verwendung von optisch aktiven Polymeren gemäß Anspruch 1 zur chromatographischen Enantiomerentrennung von (±)-trans-(E)-6-[2-(2,6-Diisopropyl-4-(4-fluorphenyl)-3-methoxymethyl-pyrid-5-yl)-ethenyl]-3,4,5,6-tetrahydro-4-hydroxy-2H-pyran-2-on.

4. Verwendung von optisch aktiven Polymeren gemäß Anspruch 1, **dadurch gekennzeichnet, daß** das optisch aktive Polymer in an Kieselgel gebundener Form eingesetzt wird.

5. Verwendung von optisch aktiven Polymeren gemäß Anspruch 4, **dadurch gekennzeichnet, daß** das optisch aktive Polymer über die Mercaptogruppen eines entsprechend modifizierten Kieselgels gebunden ist.

6. Verfahren zur chromatographischen Enantiomerentrennung von Lactonen der allgemeinen Formel (I) gemäß Anspruch 1, **dadurch gekennzeichnet, daß** man das Enantiomerengemisch unter Verwendung einer geeigneten mobilen Phase mittels eines optisch aktiven Polymers aus N-Acryloyl-S-phenylalanin-d-neomenthylamid oder aus dessen Enantiomer als chiraler stationärer Phase in die Enantiomere trennt, wobei das optisch aktive Polymer als solches, in vernetzter Form und/oder in trägergebundener Form eingesetzt wird.

7. Verfahren gemäß Anspruch 6, **dadurch gekennzeichnet, daß** man als mobile Phase ein Gemisch aus Toluol und Tetrahydrofuran verwendet.

8. Verfahren gemäß Anspruch 6, **dadurch gekennzeichnet, daß** man als stationäre Phase das optisch aktive Polymer in an Kieselgel gebundener Form einsetzt.

## Claims

1. Use of optically active polymers of N-acryloyl-S-phenylalanine d-neomenthylamide or of the enantiomer thereof as such, in crosslinked form and/or in carrier-bonded form as stationary phases for the chromatographic separation of enantiomers of lactones of the general formula (I)

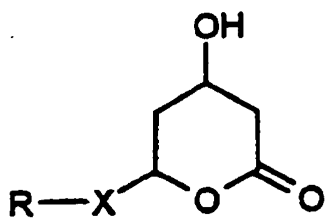

(I)

wherein

R represents an organic radical and

X represents $-CH_2-CH_2-$ or -CH =CH-.

2. Use of optically active polymers according to Claim 1 for the chromatographic separation of enantiomers of a lactone chosen from the group consisting of:

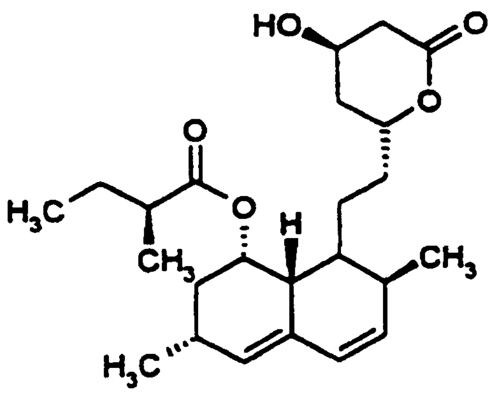

(Ia)

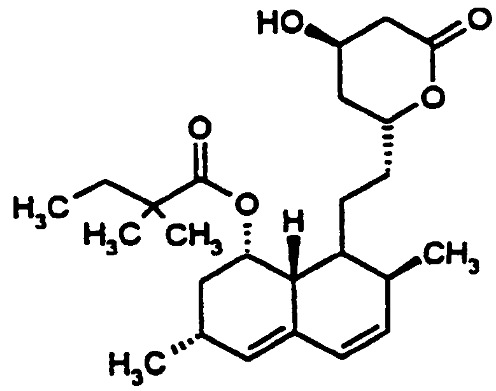

(Ib)

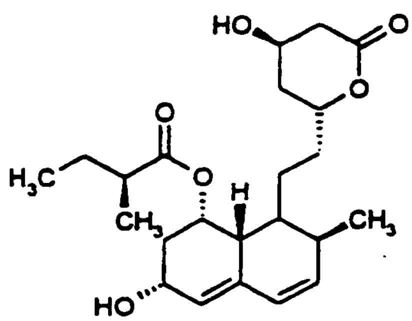

(Ic)

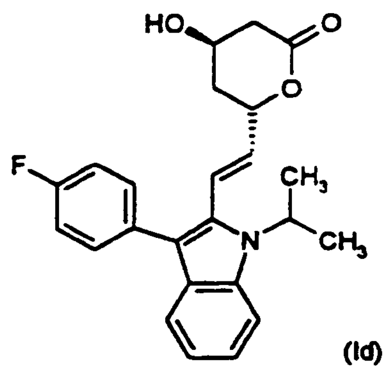

(Id)

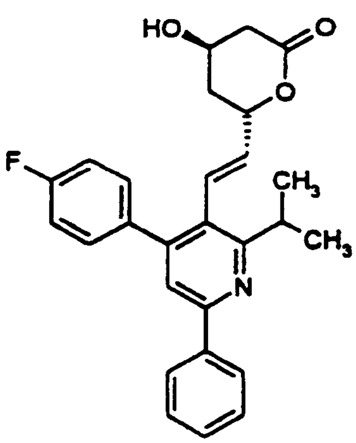

(Ie)

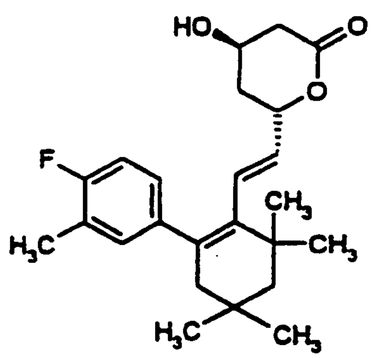

(If)

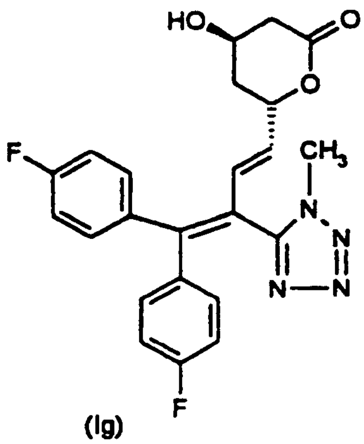

(Ig)

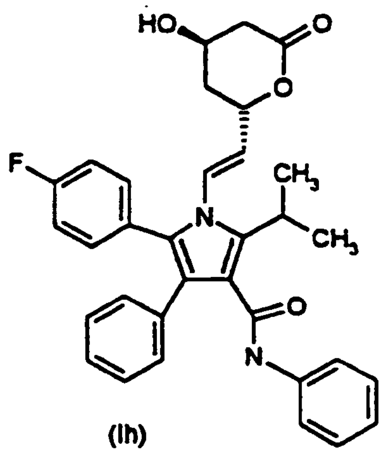

(Ih)

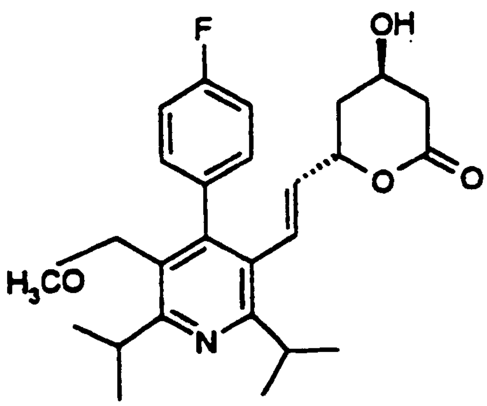

and

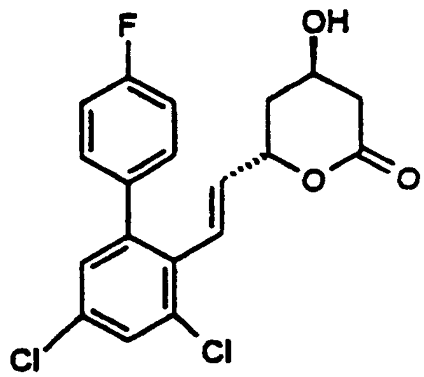

(Ii)

(Ik)

3. Use of optically active polymers according to Claim 1 for the chromatographic separation of enantiomers of (±)-trans-(E)-6-[2-(2,6-diisopropyl-4-(4-fluorophenyl)-3-methoxymethyl-pyrid-5-yl)-ethenyl]-3,4,5,6-tetrahydro-4-hydroxy-2H-pyran-2-one.

4. Use of optically active polymers according to Claim 1, **characterized in that** the optically active polymer is employed in a form bonded to silica gel.

5. Use of optically active polymers according to Claim 4, **characterized in that** the optically active polymer is bonded via the mercapto groups from a correspondingly modified silica gel.

6. Process for the chromatographic separation of enantiomers of lactones of the general formula (I) according to Claim 1, **characterized in that** the enantiomer mixture is separated into the enantiomers by means of an optically active polymer of N-acryloyl-S-phenylalanine d-neomenthylamide or of the enantiomer thereof as the chiral stationary phase, using a suitable mobile phase, the optically active polymer being employed as such, in crosslinked form and/or in carrier-bonded form.

7. Process according to Claim 6, **characterized in that** a mixture of toluene and tetrahydrofuran is used as the mobile phase.

**8.** Process according to Claim 6, **characterized in that** the optically active polymer is employed as the stationary phase in a form bonded to silica gel.

## Revendications

**1.** Utilisation de polymères optiquement actifs constitués de N-acryloyl-S-phénylalanine-d-néomenthylamide ou de son énantiomère en tant que tels, sous forme réticulée et/ou sous forme liée à un support à titre de phases stationnaires pour la séparation énantiomérique par chromatographie des lactones de formule générale (I)

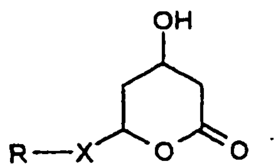

(I)

dans laquelle
R représente un radical organique et
X représente $-CH_2-CH_2$ ou -CH=CH-.

**2.** Utilisation de polymères optiquement actifs selon la revendication 1 pour la séparation énantiomérique par chromatographie d'une lactone, choisie dans le groupe constitué de :

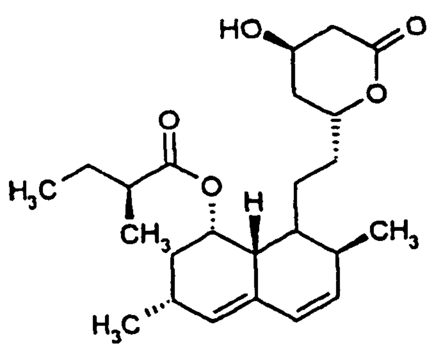

(Ia)

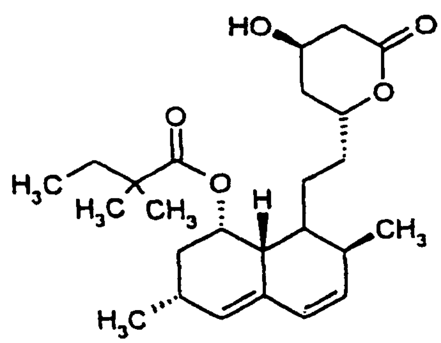

(Ib)

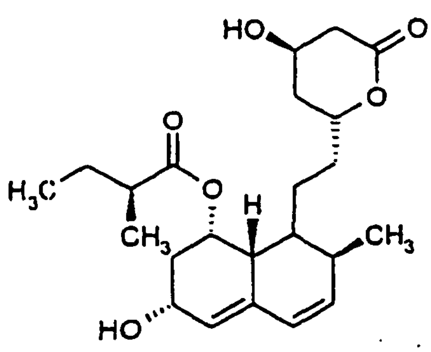

(Ic)

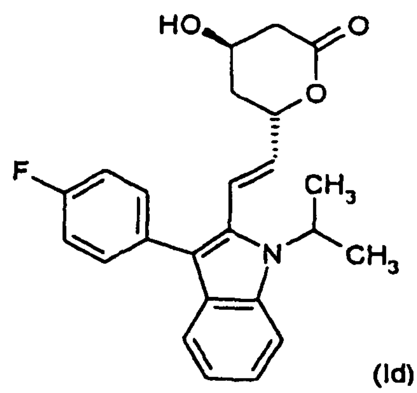

(Id)

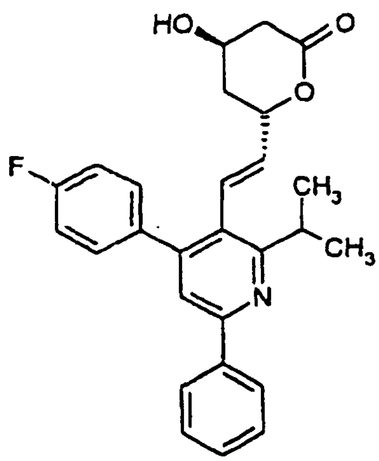

(Ie)

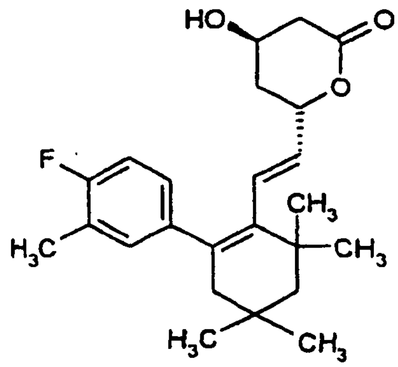

(If)

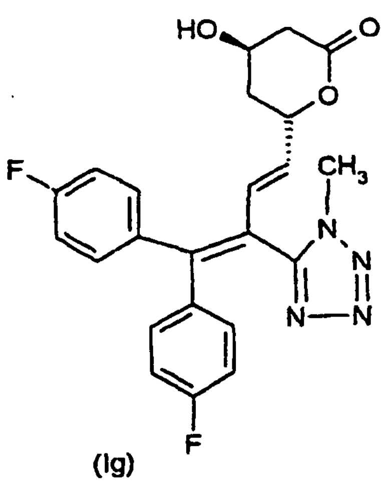

(Ig)

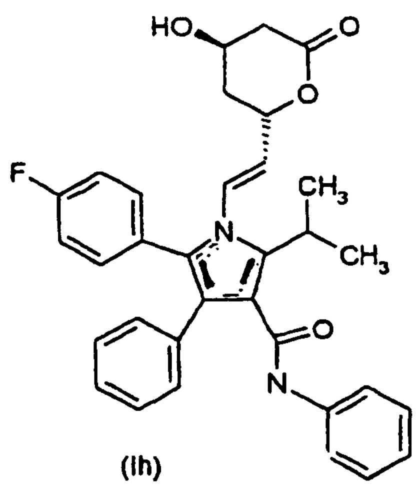

(Ih)

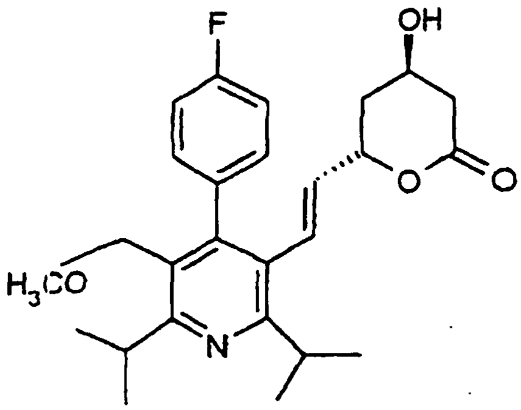

et

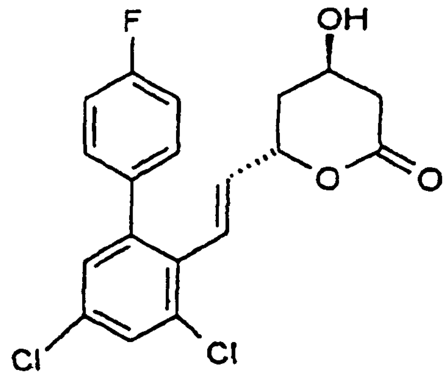

(Ii) (Ik)

3. Utilisation de polymères optiquement actifs selon la revendication 1 pour la séparation énantiomérique par chromatographie de la (±)-trans-(E)-6-[2-(2,6-diisopropyl-4-(4-fluorophényl)-3-méthoxyméthyl-pyrid-5-yl)-éthènyl]-3,4,5,6-tétrahydro-4-hydroxy-2H-pyran-2-one.

4. Utilisation de polymères optiquement actifs selon la revendication 1, **caractérisée en ce que** le polymère optiquement actif est mis en oeuvre sous forme liée au gel de silice.

5. Utilisation de polymères optiquement actifs selon la revendication 4, **caractérisée en ce que** le polymère optiquement actif est lié par le biais de groupes mercapto à un gel de silice modifié de manière correspondante.

6. Procédé pour la séparation énantiomérique par chromatographie de lactones de formule générale (I) selon la revendication 1, **caractérisé en ce que** l'on sépare le mélange d'énantiomères, en utilisant une phase mobile appropriée, au moyen d'un polymère optiquement actif constitué de N-acryloyl-S-phénylalanine-d-néomenthylamide ou de son énantiomère à titre de phase chirale stationnaire pour obtenir les énantiomères, le polymère optiquement actif étant mis en oeuvre en tant que tel, sous forme réticulée et/ou sous forme liée à un support.

7. Procédé selon la revendication 6, **caractérisé en ce que** l'on utilise comme phase mobile un mélange de toluène et de tétrahydrofurane.

8. Procédé selon la revendication 6, **caractérisé en ce que** l'on met en oeuvre à titre de phase stationnaire le polymère optiquement actif sous forme liée à un gel de silice.